# EUROPEAN PATENT APPLICATION

(11) **EP 3 666 245 A1**
(43) Date of publication of application: **17.06.2020**
(21) Application number: 17916236.7
(22) Date of filing: 08.11.2017
(51) Int. Cl.: A61H 3/00, A61F 2/62

(54) **METHOD OF ASSESSING DEGREE OF REHABILITATION USING AN ACTIVE EXOSKELETON FOR PATIENTS WITH DISORDERS OF THE MUSCULOSKELETAL SYSTEM**

(30) Priority: 30.06.2017 RU 2017123269
(71) Applicant: Obshchestvo S Ogranichennoy Otvetstvennost'yu "Exoatlet", Moscow 119121 (RU)
(72) Inventor: PISMENNAYA, Elena Valentinovna, Moscow 119192 (RU); BEREZIY, Ekaterina Sergeevna, Moscow 117421 (RU)
(74) Representative: Jeck, Anton
(86) International application number: PCT/RU2017/000838
(87) International publication number: WO 2019/004863

(57) **Abstract**

The invention relates to medicine, in particular to methods of assessing the results of therapeutic measures that have been carried out in the stages of medical rehabilitation of patients who have disorders of the musculoskeletal system. Sensors are placed between the feet of a patient and the foot supports of an active exoskeleton and measure the pressure or force at least in the heel region and the metatarsophalangeal region of the foot. Signals from the sensors are recorded for each leg during movement of the patient in the active exoskeleton for a specified time or specified number of steps at the start of a cycle and at the end of rehabilitation for a specified gait pattern. On the basis of the recorded data, rehabilitation indicators are formed and the degree of patient rehabilitation is judged according to the number of indicators fulfilled.

## Description

The invention relates to medicine, in particular to methods of assessing the results of therapeutic measures that have been carried out in the stages of medical rehabilitation of patients who have musculoskeletal disorders. It can be used for objective evaluation of the effectiveness of treatment measures course, the development of a strategy and a plan of corrective therapy when assigning the repeated cycles of rehabilitation activities.

A method to assess the effectiveness of complex rehabilitation of persons with disabilities by measuring and summing up indicators at the beginning and end of the rehabilitation course is known (RU, 2492805, A61B 5/00, 2012). It takes into account three blocks of indicators that characterize the state of the body's functions in 5 parameters: cognitive function, muscle tone and strength, severity of pain, excretory functions; activity in 5 parameters: hand capabilities, physical capabilities, mobility, level of self-service, productivity at home; participation in 4 parameters: communication skills, leisure, socialization, professional suitability.

Additionally, it is evaluated the severity of the life restrictions (SLR), which is calculated under the formula of SLR = 100 - (RP1/MRP 100) (%), where RP1 - total points on measurements results before rehabilitation, MRP - the maximum of rehabilitation potential, the maximum number of points in the assessment of functions, activity and participation. When the coefficient SLR is within 10-24% slight degree of severity living life is determined, when SLR is within 25-49% -moderate degree, when SLR is within 50-74% - expressed degree and at the value of SLR 75-100% - significantly expressed degree.

Then the effectiveness of rehabilitation is calculated under the formula ER = (RP2 - RP1)/MRP·100 (%), where RP1 - the total points before rehabilitation, RP2 - the total points after rehabilitation, MRP -the maximum of rehabilitation potential, the maximum number of points in the assessment of functions, activity and participation.

The result is evaluated as a percentage, taking into account the SLR coefficient. If the coefficient SLR is within 10-24%, then the effectiveness of rehabilitation within 15-25% is defined as very good, 10-14% - good, 5-9% - satisfactory, 0-4% - without dynamics; if the coefficient SLR is within 25-49%, then rehabilitation effectiveness within 25-50% is estimated as very good, 15- 24% - good, 5-14% - satisfactory, 0-4% - without dynamics; if the coefficient SLR is within 50-74%, then efficiency of rehabilitation within 20-35% is assessed as very good, 10-19% - good, 5-9% - satisfactory, 0-4% - without dynamics; if the coefficient SLR is within 75-100%, then rehabilitation effectiveness 10-15% is assessed as very good, 5-9% - good, 1-4% - satisfactory, 0-0.09% - without dynamics.

It is known the method of assessing the functional condition of patients with lower limb joint pathology during rehabilitation by examining clinical- functional signs before and after treatment (RU,2233619, A61B 5/11, 2003), according to which the study is carried out by measuring the following characteristics: the maximum number of times a patient can get up from a high chair; the lowest height of the chair from which the patient can stand up or sit on; the height of the step on which the foot can stand, for each leg separately; the time of standing on the leg, in seconds, for each leg separately.

After that, the initial value of each trait is compared with the degree of its increase in percent, while the first sign gets 1 point with an increase in the value of this sign by 50% - at the initial value of up to 20 times, by 25% - at the initial value of 20 to 40 times and any increase - at the initial value of more than 40 times. The second and the third traits get one or more points when moving from one height of support to another. The fourth trait, respectively, gets one point when increased by 50% - at its initial value of up to 30 s, by 25% - at the initial value in the range from 30 sec to 60 sec and at any increase - with an initial value of more than 60 sec.

Then the number of points is added up. If the number of points is equal to 1 the dynamics of the patient's condition is assessed as "slight improvement", with a score of 2-4, the dynamics of the patient's condition is assessed as "improving," and with a score of 5-6 conclude to a significant improvement of the functional state of the patient.

The known methods of assessing the degree of rehabilitation are not sufficiently accurate and informative. In addition, they are not suitable for assessing the rehabilitation of patients with partial or complete loss of musculoskeletal functions when using movement assistance devices, in particular active exoskeletons, as a means of rehabilitation.

The use of active exoskeletons, progress in the development and production of which has recently been noted in technologically advanced countries, allows the rehabilitation of patients with musculoskeletal disorders in conditions as close as possible to natural movements, in particular gait, of a person. At the same time, there is a need to develop new, suitable for this case, instrumental ways to assess the degree of rehabilitation on physical indicators, giving an objective picture of the process of restoring the patient's health.

The technical result of the proposed invention is to expand the arsenal of technical means for assessing the degree of rehabilitation of patients with musculoskeletal disorders. In addition, the technical result is also to obtain reliable data on the course of rehabilitation and improve the accuracy of the assessment.

This technical result is achieved due to the fact that the method of assessing the degree of rehabilitation using an active exoskeleton in patients with musculoskeletal disorders is carried out as follows. Sensors measuring pressure or force, at least in the heel region and metatarsophalangeal region of the foot, are placed between the patient's feet and the foot supports of the active exoskeleton. Signals from the sensors are recorded for each leg during movement of the patient in the active exoskeleton for a specified time or specified number of steps at the start of a cycle and at the end of rehabilitation for a specified gait pattern.

As will be shown below, before conducting a rehabilitation course, the patient must be trained to use an active exoskeleton to the degree of control or completely independent, or with external assistance, depending on his clinical condition. Naturally, each patient will have their own degree of training.

Based on this, and taking into account the selected pattern of movement of the active exoskeleton, the attending doctor selects the necessary number of double steps or time sufficient to record the parameters under investigation. The minimum necessary to get a real picture of the state of the rehabilitation process is to perform 10 double steps.

At the end of the rehabilitation cycle or after a predetermined number of rehabilitation sessions with a predetermined gait pattern, a second recording of sensor signals is performed when the patient moves in an active exoskeleton for a predetermined time or a predetermined number of steps.

Depending on the diagnosis of the disease, the patient's physical condition, and various socio-economic reasons, the duration of the rehabilitation cycle may vary greatly in each case. Usually, rehabilitation using an active exoskeleton takes two to three weeks or, respectively, 10 to 15 sessions. However, with a long rehabilitation cycle, for example, for 2 months, the attending doctor must receive objective information about its course. Therefore, an assessment can be made, for example, every three weeks of the course.

Then, based on the recorded data, the first and second time characteristics of the locomotor cycle are determined and these characteristics are compared with each other and with the time characteristics obtained for a healthy person on this type of active exoskeleton with a predetermined gait pattern.

Time characteristics are generally accepted parameters of the locomotor cycle, such as duration of double step, the interval of support on the heel, the interval of support on the foot, the interval of support on the toe, swing phase, double-support phase, shift, rhythm coefficient. (see A.S.Vitenson, K.A.Petrushanskaya. From natural to artificial control of locomotion, p.115, Scientific-medical company MBN, Moscow, 2003).

Based on the recorded data for each leg, the first and second averaged characteristics of the locomotor cycle are determined as a function of the vertical component of the support reaction over (relatively) time, or as a function of the ratio of the vertical component of the support reaction to the patient's weight over (relatively) time at the support phase.

In addition, based on the recorded data for each leg, the first and second distributions of pressure under the foot in the middle of the stance phase are determined.

The following indicators are used for assessing the degree of rehabilitation:
- changing the time characteristics of the locomotor cycle in the direction of time characteristics for a healthy person,
- increasing the amplitude of the average ratio of the vertical component of the support reaction to the patient's weight or the average vertical component of the support reaction for both legs,
- alignment of the amplitudes of the averaged ratio of the vertical component of support reactions to the weight of the patient, or the average of the vertical component of support reactions for both legs,
- detecting three pronounced extremes corresponding to the heel-strike, the push-off and the median minimum for the second averaged characteristics for both legs,
- increasing the contact spot in the lateral and metatarsophalangeal parts of the second pressure distribution compared with the first.

Rehabilitation is assessed as satisfactory if one or two indicators are present (identified), rehabilitation is assessed as good if three or four indicators are present, and rehabilitation is assessed as excellent if there are more than four indicators.

As will be shown below, the most preferable implementation is a method in which measuring insoles are used as pressure or force sensors. However, in general, even if the accuracy and reliability of measurements are reduced, it is sufficient to have one or two pressure or force sensors located in each heel and metatarsophalangeal region (parts) of the foot.

The above is a summary of the essence of the invention and, therefore, may contain simplifications, generalizations, inclusions and/or exclusions of details; therefore, specialists in this field of technology should take into account that this summary of the essence of the invention is only illustrative and does not imply any limitation.

For a better understanding of the essence of the proposed invention, a description of a specific implementation example is provided below, which is not a restrictive example of the practical implementation of a method for assessing the degree of rehabilitation in accordance with the claimed invention, with reference to the drawings, which are presented next.

Fig.1 shows the dynamic characteristics and distribution of pressure under the foot in a healthy person.

Fig.2 shows the dynamic characteristics and distribution of pressure under the foot of the patient before and after rehabilitation.

Fig.3 shows the distribution of pressure under the foot of the patient before and after rehabilitation and the two-humped shape of the curve of the averaged ratio of the vertical component of the support response to the patient's weight.

Fig.4 shows graphs of the ratio of the vertical component of the support reaction to body weight, which have several minimums, in patients at the beginning of the rehabilitation course.

Fig.5 shows graphs of the average ratio of the vertical component of the support reaction to the patient's weight after rehabilitation.

In the rehabilitation of patients with musculoskeletal disorders using an active exoskeleton, walking takes place in an exoskeleton worn by the patient, which consists of a pelvic, two femoral, two links of the lower leg and two feet, as well as two ankle joints that provide rotation of the feet in the sagittal plane.

The movements of the links in the exoskeleton are performed in the sagittal plane. The exoskeleton is equipped with drives in the hip and knee joints that provide relative movement of the corresponding links. The exoskeleton includes sensors that determine the inter-link angles of the hip and knee joints, as well as sensors for determining the strength of the support reaction, located in the foot insoles. Information from these sensors is collected and processed by a computer system that transmits information wirelessly to the research computer. The exoskeleton is attached to the patient by means of straps that hold the patient's torso, placed in a medical corset if necessary. Thighs and tibias are also held by straps.

When using an exoskeleton during walking, it affects the patient at various points of the body, moving the transferred leg along a set program trajectory in accordance with the selected pattern. In general, the person and the exoskeleton integrated with it are a human-machine system. Interaction of a person and an exoskeleton is carried out by means of forces arising in the places of attachment of a person with an exoskeleton (straps and boots).

It is not yet possible to measure the forces in the straps. However, the degree of load on the patient's feet can be measured using force sensors under the operator's foot. So, if the anthropometric parameters of a person and an exoskeleton correspond to each other at rest, the vertical reaction force in the operator's feet is determined by the weight of the person himself. At the same time, part of the weight falls on crutches. Therefore, flexible force sensors from Tekscan were used to assess the load of the patient's feet in the exoskeleton when walking on a flat horizontal surface. These sensors must be placed in the boot between the person's foot and the support pad of the exoskeleton.

The use of insoles allows us to explore the following biomechanical parameters of walking: basic (velocity, cadence, double step length, the duration of the locomotor cycle), time (duration stance phase, swing and double-support phases, the rhythm coefficient), dynamic parameters (vertical component RZ of the main vector of support reaction) and the pressure distribution under the foot.

The advantage of using insoles in comparison with traditional dynamometry is the ability to register the vertical component RZ of the support reaction in each step, which allows us to get data on a variety of steps. The study of a large number of support reactions allows to conduct detailed processing of the results and obtain a coefficient of variability - a necessary parameter for assessing the degree of development of the exoskeleton, analyze changes in stability and support capacity as the patient adapts to walking in an exoskeleton, track the decrease in the time of support on crutches.

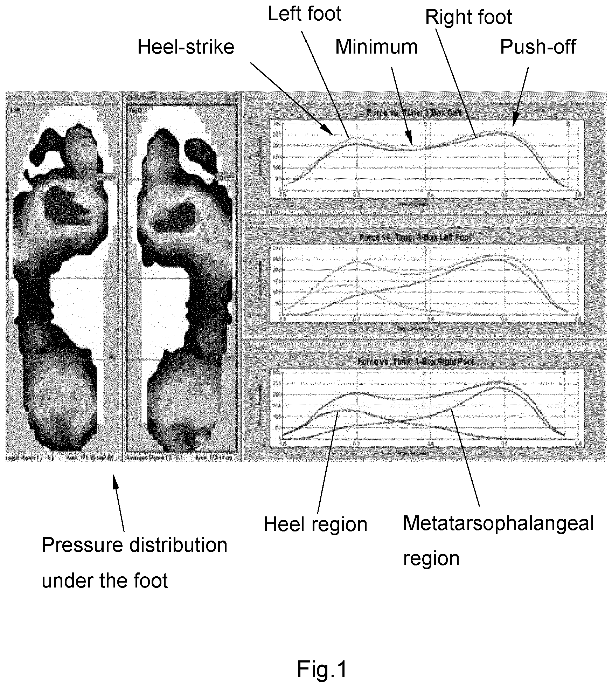

The measuring system of force interaction allows you to obtain dynamic data in real time in the form of a color picture (Fig. 1), which characterizes the level of force load on various parts (region) of the feet of both legs (figure on the left). Measurements are performed with a high frequency of sensor polling (up to 6000 Hz). The sensor system allows calibration in various modes (at rest and when walking). It also allows you to display the measurement results on graphs (Fig.1 on the right) or in the form of arrays of numerical data.

Fig.1 shows the dynamic characteristics and distribution of pressure under the foot in a healthy person. Clearly visible is the two-humped shape of the vertical component of the support reaction RZ with pronounced extremes corresponding to the heel-strike, the median minimum and the push-off.

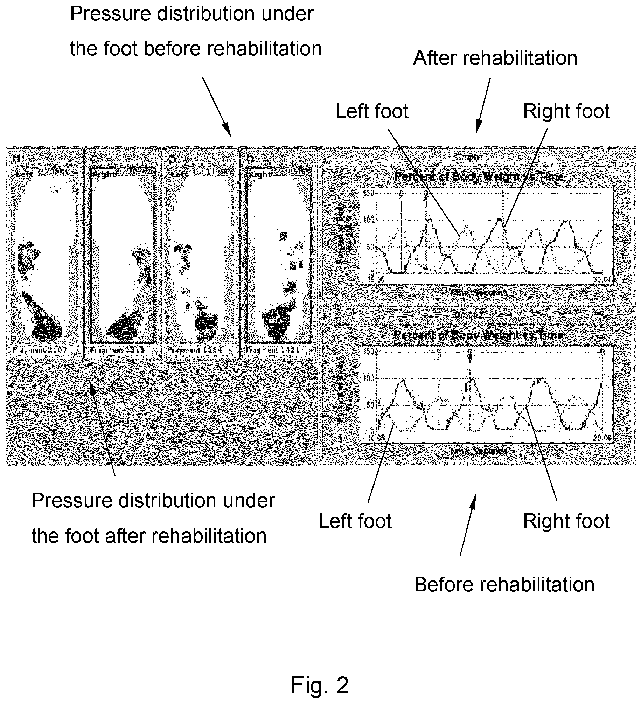

Fig.2 shows the dynamic characteristics and distribution of pressure under the foot of the patient before and after rehabilitation. It can be seen that the vertical component RZ of the support reaction (in relation to body weight) before rehabilitation has a pronounced trapezoidal shape. In this case, the amplitude of the vertical component RZ of the support reaction of the left leg is significantly less than the amplitude of the vertical component RZ of the support reaction of the right leg. The distribution of pressure under the patient's foot, measured in the middle of the stance phase, before rehabilitation shows that the patient's support is mainly performed on the heel.

After the rehabilitation course, the amplitude of the vertical component RZ of the support reaction for the left leg significantly increases and almost equals the amplitude for the right leg, although the shape does not change. The distribution of pressure under the patient's foot shows that the patient has a support on the lateral part of the foot.

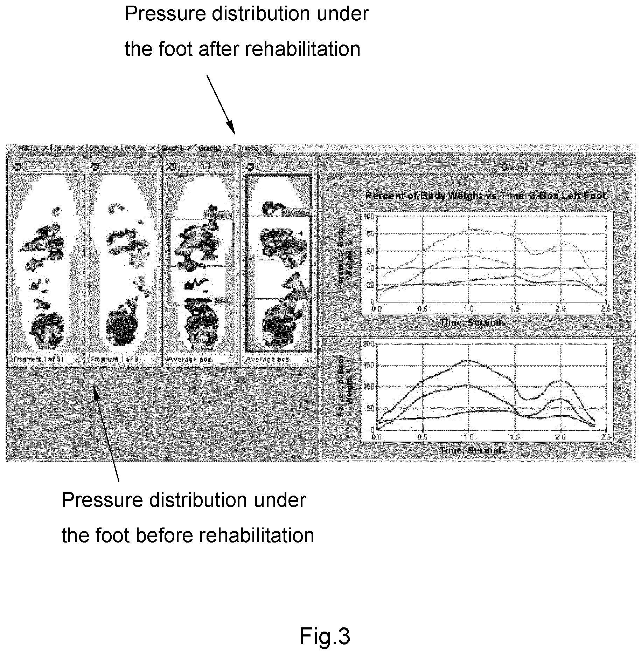

Fig.3 shows the distribution of pressure under the foot of the patient before and after rehabilitation. It can be seen that after rehabilitation the contact spot of the pressure distribution under the foot increases in the metatarsophalangeal region of the foot. Also it is shown the two-humped shape of the curve of the averaged vertical component RZ of the support reaction (in relation to the patient's weight) obtained after a course of rehabilitation.

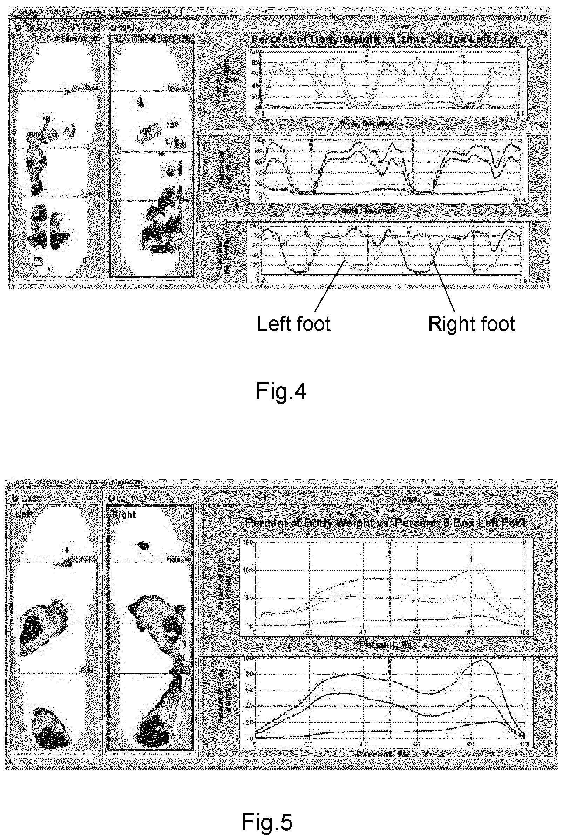

Fig.4 shows graphs of the vertical component RZ of the support reaction (relative to the patient's weight), which have several minima, in patients at the beginning of the rehabilitation course. After the rehabilitation course, as shown in Fig.5, the graphs of the averaged vertical component RZ of the support reaction (in relation to the patient's weight) acquire a pronounced two-humped shape with extremes corresponding to the heel-strike, the median minimum and the push-off.

Example. Studies were conducted on 5 healthy persons and 5 patients with the effects of spinal cord injury when walking in an exoskeleton "ExoAtlet". Studies of healthy persons and patients were conducted only after learning them to walk in an exoskeleton for several days.

Studies have shown that when walking in an exoskeleton in healthy persons, the duration of the locomotor cycle is 3 seconds, the length of the double step is 0,66 m, the walking cadence is 40 steps/min, and the average walking velocity is 0,22 m/s. In this case, the duration of the stance phase on the left leg is 71.0%, and the duration of the swing phase is 29.0%; on the right leg, this number is 69,3% and 30,7%, respectively. Thus, there is a certain asymmetry in the time parameters of walking even when walking healthy persons in an exoskeleton. The lower limb that has a shorter duration of the swing phase, i.e. the left leg, is more secure. But the greatest degree of asymmetry is shown in the duration of the double-support phase. For the left leg, the duration of the double-support phase is 18% of the locomotor cycle, and for the right - 22,3%.

It should be noted that when walking in an exoskeleton, crutches are used, and the transfer of crutches and support on them requires additional time, especially at the beginning of training. An increase in the duration of the double-support phase is associated with a longer rearrangement of crutches and supporting on them during this phase of the step. When walking healthy persons in an exoskeleton, it is possible to distinguish the phase of support on the forefoot. The duration of this phase is 9,6% of the locomotor cycle on the left leg, and 10,4% of the cycle on the right leg.

The presence of the toe support phase makes it possible to calculate another very important time parameter of walking - the interval T. This interval is determined by the time between the separation of the heel of one foot from the support surface and the moment when the other foot steps on the support. The interval T even in healthy persons when walking in an exoskeleton takes a negative value, namely - 12,7% on the left leg and -7,6% on the right leg. A negative value of the interval T means that the heel separation occurs only when the other foot is already firmly on the support.

The most interesting is the transformation of the dynamic parameters of walking, namely the vertical component RZ of the support reaction. When walking healthy persons in an exoskeleton, the vertical component of the support reaction retains the traditional two-humped shape. However, the heel- strike is implemented at the level of 15% of the cycle, then there is a long transition to the minimum, its extreme value falls on 39% of the locomotive cycle, and then a very short phase of the push-off, which is implemented at the level of 50% of the cycle. Extreme values of the vertical component do not reach the level of body weight, the values of the heel-strike and the push-off are approximately the same and is 94%, and the minimum - 84%.

A characteristic feature when walking healthy trained people in an exoskeleton is the fact that, as with walking without an exoskeleton, the minimum of RZ curve corresponds to the moment when the transferred lower limb is opposite the support one, and the overall center of mass (OCM) occupies the highest position. In this case, the minimum vertical component of one leg occurs in the single-support phase, i.e. during the swing phase of the contralateral leg.

Rehabilitation studies in 5 patients with the consequences of spinal cord injury show that the duration of the locomotor cycle when walking in an exoskeleton does not change in comparison with the norm and is also equal to 3 seconds, but the step length decreases by 15% (0,56 m), and the walking velocity - by 14% with the norm. At the same time, the duration of the stance phase significantly increases on both legs, on the left leg by 12% (79,4%), on the right leg by 17% (80,9%).

The duration of the swing phase when walking in an exoskeleton is significantly reduced compared to the norm: on the left leg by 29% (20,6%), on the right leg by 38% (19,1%). There is also a significant increase in the duration of the double-support phase, especially on the left leg - by 68% (30,2%) and by 35% - on the right leg (30,1%). In patients with the consequences of spinal cord injury, it is not possible to distinguish the toe support phase, and, consequently, the interval T, before the start of training.

Before the course of rehabilitation in the exoskeleton, the curve of the vertical component Rz of the support response in patients with the consequences of spinal cord injury has a trapezoidal shape, but there is a sharp shift of all extreme values to the right along the time axis. According to averaged data, the first vertex of the curve Rz - heel-strike - tightened dramatically and is projected onto the x-axis in the region t=34% of cycle, minimum - in the region t=59%, push-off - in t=69%. All extreme values of the Rz curve are sharply reduced compared to the corresponding values when walking healthy people in an exoskeleton. Compared with healthy persons, the value of the heel-strike was reduced on the left leg by 47% (49,6%), the minimum - by 65%

(29,7), and the push-off - by 45% (51,6%). On the right leg the decrease in these parameters is respectively 19% (76,2%), 52% (40,6%) and 22% (73,4%). Thus, in patients with the consequences of spinal cord injury when walking in an exoskeleton, there is a clear asymmetry of dynamic parameters.

The obtained data allow us to conclude that in patients with the consequences of spinal cord injury when walking in an exoskeleton, there is a distinct change in its basic, time and dynamic parameters in comparison with the corresponding parameters of walking in healthy persons, manifested in a decrease in the length of the double step, the average velocity of movement, and the transformation of the time structure of the step - increasing the duration of the stance and double-support phases and decreasing the duration of the swing phase, absence of interval T, changing of the Rz curve shape, in a sharp decrease in the magnitude of extreme values of the vertical component of support reactions and their move to the right along the time axis.

After a course of walking training in the exoskeleton slight changes in the time structure of the step are shown. On the left leg, there is a slight decrease in the duration of the swing phase, but it is also unreliable. Thus, the time structure of walking in patients with the consequences of spinal cord injury remains very stable and changes a little even after a course of complex rehabilitation, including training walking in an exoskeleton in combination with electrical stimulation of the spinal cord.

After a 10-day course of walking training in the exoskeleton, there is a change in the shape of the vertical component of the support reaction on both legs. The vertical component acquires a distinct two-humped shape with clearly defined maxima. In this case, dynamic asymmetry practically disappears, i.e. the value of extreme parameters becomes approximately the same on both legs. There is a sharp increase in all extreme values of support reactions, the value of the heel-strike and the push-off on both legs exceeds the body weight. On the left leg, the value of the heel-strike increases approximately 2,5 times (121,4%), the value of the minimum - 2,6 times (77,9%), the value of the push- off - 2,1 times (109,8%). On the right leg, the increase is 64% (125,2%) for the heel-strike, 107% (83,9%) for the minimum, and 58% (116,2%) for the push-off. It can be assumed that an increase in the support capacity of a weaker lower limb contributes to an increase in the push function of a less affected one.

The obtained results indicate a high efficiency of rehabilitation. The main effect of the rehabilitation is to significantly improve dynamic parameters of walking, namely, to change the shape of the Rz curve i.e. the appearance of double-humped curve instead of trapezoidal, the shift of all extreme values to the left along the time axis, a significant increase in the magnitude of the heelstrike and the push-off on both legs, reducing the time supporting on the crutches. The fact that the value of the heel-strike and the push-off exceeds body weight indicates a high degree of exoskeleton mastery, and an increase in the supporting and pushing functions of the lower limbs.

## Claims

1. Method for assessing the degree of rehabilitation using an active exoskeleton in patients with musculoskeletal disorders, consisting in the following
- sensors measuring pressure or force, at least in the heel region and metatarsophalangeal region of the foot, are placed between the patient's feet and the foot supports of the active exoskeleton,
- the first recording of sensor signals for each leg are performed when the patient moves in an active exoskeleton during a predetermined time or for a predetermined number of steps at the beginning of the rehabilitation cycle with a predetermined gait pattern,
- the second recording of sensor signals are performed when the patient moves in an active exoskeleton for a predetermined time or a predetermined number of steps at the end of the rehabilitation cycle or through a predetermined number of rehabilitation sessions in a predetermined gait pattern,
- based on the recorded data, the first and second time characteristics of the locomotor cycle are determined and compared with each other and with the time characteristics obtained for a healthy person on this type of active exoskeleton in a predetermined gait pattern,
- based on the recorded data for each leg, the first and second averaged characteristics of the locomotor cycle are determined as a function of the vertical component of the support reaction relatively time or as a function of the ratio of the vertical component of the support reaction to the patient's weight relatively the stance phase time,
- the first and second pressure distributions under the foot in the middle of the stance phase for each leg are determined,
- the following assessment indicators are used:
- changing the time characteristics of the locomotor cycle in the direction of time characteristics for a healthy person,
- increasing the amplitude of the average ratio of the vertical component of the support reaction to the patient's weight or the average vertical component of the support reaction for both legs,
- alignment the amplitudes of the average ratio of the vertical component of the support reaction to the patient's weight or the average vertical component of the support reaction of both legs,
- identification of three pronounced extremes corresponding to the heel- strike, the push-off and the median minimum for the second averaged characteristics for both legs,
- increasing of the contact spot in the lateral and metatarsophalangeal parts for the second pressure distribution compared to the first,
- rehabilitation is assessed as satisfactory if one or two indicators are present (detected),
- rehabilitation is assessed as good if there are three or four indicators,
- rehabilitation is assessed as excellent if there are more than four indicators.

2. The method of rehabilitation according to claim 1, **characterized in that** a measuring insoles are used as pressure or force sensors.
